Europäisches Patentamt

European Patent Office

Office européen des brevets

(19)

(11) Publication number: **0 231 729 B1**

(12) **EUROPEAN PATENT SPECIFICATION**

(45) Date of publication of patent specification: **04.08.93**

(51) Int. Cl.5: **C12P 19/14**, C12P 19/22, C13K 1/06

(21) Application number: **86850418.4**

(22) Date of filing: **02.12.86**

(54) **Process for the enzymatic degradation of whole flour of carbohydrates to produce a foodstuff, the foodstuff and its use.**

(30) Priority: **06.12.85 SE 8505783**

(43) Date of publication of application:
**12.08.87 Bulletin 87/33**

(45) Publication of the grant of the patent:
**04.08.93 Bulletin 93/31**

(84) Designated Contracting States:
**AT BE CH DE ES FR GB GR IT LI LU NL SE**

(56) References cited:
**EP-A- 0 134 546**
**DE-B- 911 723**
**GB-A- 1 426 997**
**US-A- 4 217 414**

**Patent Abstracts of Japan, vol. 6, no. 57(C98)**

**Chemical Abstracts, vol. 98, 1983, abstract no.55945r**

**Patent Abstracts of Japan, vol. 6, no. 266 (C142)**

(73) Proprietor: **HERWOOD N.V.**
**De Ruyterkade 62 P.O. Box 812**
**Curacao(AN)**

(72) Inventor: **Bergkvist, Rolf Ragnar**
**Alnarpsvägen 9**
**S-232 02 Akarp(SE)**
Inventor: **Claesson, Karl Olov**
**Slavsta**
**S-755 90 Uppsala(SE)**

(74) Representative: **Inger, Lars Ulf Bosson**
**L + U INGER Patentbyra AB Garvaregatan 12**
**S-262 63 Ängelholm (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

**Description**

The present invention relates to the manufacture of nutritionally and functionally valuable products by enzymatic degradation of the carbohydrates of cereals and other starch-containing products.

Processes have been developed for the isolation of the main components, starch and protein, from such raw materials. Starch produced in a pure form is used directly or for the manufacture of starch syrup or glucose by hydrolysis with acid and/or enzymes. The proteins are isolated for special use, and from wheat native as well as devitalized gluten is isolated.

Processes have also been patented, wherein the starch as well as the proteins are degraded simultaneously or in any order by enzymatic treatment of the raw material. These processes involve an uncontrolled interaction between the enzymes resulting in varying composition of the final product. Moreover, the proteolytic enzymes used attack not only the proteins of the raw material but also to a varying extent the enzymes used in the process, which results in reduced enzymatic activity and further difficulties to control the process towards a well defined and uniform final product.

The proteins are degraded to a varying extent to peptides and free amino acids resulting in varying taste and colour of the final product. Furthermore, some of the free amino acids produce a bitter taste unacceptable for many areas of use.

The nutritional value of the proteins is negatively affected by the Maillard-reactions which inevitably take place, amino acids having free amino groups reacting with reducing types of sugars under the formation of brown, uncharacterized polymers, melanoids. Some of these products have positive value as taste or coloured donors, whereas other are suspective of being carcinogenic or mutagenic. Intense research is presently made concerning the Maillardcompounds, and it is fully clear that high concentrations thereof should be avoided in food-stuffs.

Conventional processes for the isolation of starch involve complicated procedures in which i.a. large quantities of water are used. Biproducts of inferior market value are obtained as biproducts, as well as sewage water difficult to handle.

According to the present invention there is used as raw material flour of grains of wheat, barley, rye, oat, rice, maize, sorghum and other starch-containing grains, ground to the desired particle size. The fibre contents is adapted by dry-fractionation to the concentration desired in the final product.

Conventional and well developed processes are used for flour manufacture, and the raw material supply is abundant since such raw materials have wide-spread use in other areas. The fibre fraction can be separated by a sifting procedure, and since this product already has a market it can be disposed of without difficulties. In wet fractionation used in other connections there will be obtained a fibre fraction which has to be separated and dried, something which negatively affects the process economy. This latter process has been applied in several of the processes previoulsy patented.

A fundamental idea of the present invention is that the desired quantity of fibre as well as proteins and other valuable components of grain are present in the raw materials and are also found in the final product. It is only the carbohydrates and primarily the starch that is subject to enzymatic degradation.

Starting from this basic concept there is thus provided by the present invention a process for the manufacture of food-stuff products starting from whole flour of starch-containing cereal grains. In accordance with the invention the whole flour contents of carbohydrates are enzymatically degraded while the other components of the whole flour are maintained substantially intact. The enzymatic degradation of the carbohydrate contents of the whole flour preferably takes place while using a starch hydrolizing enzyme. This enzyme is suitably selected among alfa and beta amylases.

Even if the technique according to the invention is applicable on any grinding product of cereals and other starch-containing products the invention is primarily applicable to whole flour originating from grains of wheat, barley, oat, rye, rice, maize and sorghum. The enzymatic degradation is suitably carried down to at least oligosaccharide level and possibly also at least partly to mono- or disaccharide level. By the term "oligosaccharide" there is meant carbohydrate fractions containing up to about 10 monomer entities.

The invention is particularly applicable to the treatment of whole flour originating from the four basic cereals, namely wheat, barley, rye and oats.

The enzymatic treatment can be carried out in two steps with an initial treatment with an alfa-amylase and a subsequent treatment with a beta-amylase, said subsequent treatment possibly taking place in combination with an enzyme of pullalanase type. The enzymatic treatment may, in accordance with one aspect of the invention, be carried to at least partial formation of glucose which then, to provide for increased sweetness of the product, is at least partly converted to fructose by treatment with the enzyme glucose isomerase.

According to an alternative embodiment of the invention the enzymatic degradation mainly takes place to the formation of maltose and maltotriose.

The invention also relates to the use of food-stuff products manufactured by the process as substitute for saccharose or saccharose-based sweeteners in digestable products of different types. Among such products there may be mentioned bakery products, nutritional drinks, desserts, confectionaries, jams, soft drinks, marmalades etc. The food-stuff products manufactured according to the invention is particularly suited for use in the baking of ordinary bread as a replacement for the normal sweetener, for example saccharose, in a pure form or as a component of syrup or the like.

When applying the technique of the present invention the proteins may be denaturated to a varying extent but they are not decomposed to smaller peptides or free amino acids, the risk for Maillard-reactions being minimized.

Since all components of the raw material is found also in the final product the yield from the process is almost 100% and no valuable substances are lost. No sewage water or other environmental problems are created.

The process according to the present invention is adapted in dependance of the use of the final product and different alternative procedures have been developed:

1) The enzymatic degradation of the carbohydrates is adapted by the selection of enzymes and incubation conditions in relation to the raw material used and the desired composition of the final product. In certain cases there will be desired a high contents of mono- and/or disaccharides, whereas in other cases di-, tri-, tetra- and higher polymers of glucose are desirable.

2) The final product which contains soluble as well as insoluble components can be used directly in many areas, i.a. in bakeries. Expensive concentration or drying can be avoided and is not necessary since additional water must be added anyway in the subsequent use.

If longer stability is desirable the final product can be pasturized and/or packaged in such a manner as to avoid infection. The choice of storage conditions also affects stability and even deep-freeze storage is conceivable. Addition of a suitable preservative is another alternative for increasing stability.

3) The final product may also be evaporated to a dry substance contents of 65-83% resulting in increased stability.

If the contents of glucose does not exceed 20% the final product may also be completely dried using conventional hot air processes. At higher glucose contents the product becomes hygroscopic to such extent that it must be protected from moisture in a dry form.

4) In those cases where a clear syrup is desirable the insoluble components, mainly fibres and proteins, can be removed by centrifugation after the enzymatic process has been concluded and the enzymes inactivated.

The fibre-protein fraction can be used directly or after drying. The soluble fraction containing i.a. the soluble carbohydrates can be used either directly or after evaporation to a syrup or after drying to a powder to the extent the composition so permits.

5) If a sweeter final product is desirable part of the glucose contents of the product can be converted into fructose by enzymatic isomerisation. The addition of Aspartame, fructose or other sweet components can also be made in order to obtain an even sweeter final product.

The starch of the raw materials contains as building elements only glucose, whereby the degradation products consist of glucose polymers of varying size down to the smallest building entities.

The enzymatic decomposition can be controlled so that the final product obtains optimum composition with regard to the area of use. In this context also the role of the carbohydrates established by the research of recent years has been taken into consideration.

The composition of the final product is motivated i.a. by the following important properties of specific carbohydrates:

A) Biological carbohydrates

Recent research has clearly shown that carbohydrates not only are energy donors but also play an essential role in many physiological contexts, not least by the establishment of bacterial infections.

Most infections attacking us are created in connection with the mucous membranes. The capacity of bacteria to bind selectively to the epithelium of the organs where the infection is created has been found to be a decisive virulence factor of the bacterium.

The research during the last five years has shown that the presence of "attachment sites", receptors, at the epithelium surfaces influences in a decisive manner the susceptibility for infections. In several cases the receptors have been found to have as an active structural element a simple oligosaccharide.

As an example there may be mentioned that the receptor for certain europathogenic E.coli has been found to be simple disaccharide, galoctose 1-4 galactose. The presence of this structure on the epithelium of the urinary tracts is a basic requirement in order that the bacteria shall stay on the site, multiply and climb up through the urinary tract to the kidney and there live on and cause often serious kidney damage.

Similar receptors have been identified for a number of other bacteria, and there are clear indications that carbohydrate receptors play an important role also in more normal physiological systems.

Infections in the oral cavity as well as the intestinal tract are dependent on the the presence of active receptors. If receptor analogues are supplied through the food these may compete with the own receptors of the mucous membranes concerning the adhesion of the bacteria thereby exerting an inhibiting or therapeutic effect.

Active receptors are present not only in the form of simple oligosaccharides but are also parts as structural elements in larger polysaccharides. In partial enzymatic decomposition of polysaccharides, for example starch, such active structural elements will also appear or be exposed.

Within the scope of the present invention it has been found that the decomposition products of starch produced in accordance with the invention have a positive effect on serious intestinal infections in babies as well as pig and chicken. The effect can be derived from the presence of receptors among the decomposition products, said receptors resulting in alleviating the infection as well as providing for a general improvement of growth.

In laboratory experiments an agglutinating effect against Streptococcus mutans of several specific degraded fractions of starch in wheat flour and grains has been established. This also shows in this case the presence of active receptors against the oral bacterium of serious importance in connection with caries.

In this context it is important that the starch is degraded only partially and not fully to the simplest building stones, maltose and glucose.

In view of the above-mentioned important properties partially decomposed starch will obtain an increasing use in for example "nutritional drinks" for individuals of varying age and as "medical foods" and other uses. By admixing with milk or milk powder there will be obtained nutritionally high-value products of a clearly antibacterial effect and having at the same time a lower contents of lactose which is of great importance for the fraction of the world population suffering from lactose intolerance. Also drinks and other products based on soya can advantageously be supplemented with such "active starch products".

B) Cariologically advantageous carbohydrates

According to the above there may be obtained by partial degradation of starch receptor structures active to oral bacteria making it possible to remove said bacteria from the oral cavity.

It is equally essential from a caries prevention point of view to minimize the intake of saccharose by replacement in the food thereof with the substitutes for saccharose that can be manufactured by the processes described in the present invention.

The primary cause for caries is the presence of Streptococcus mutans in the oral cavity. This bacterium forms in the presence of saccharose deposits, plaque, on the surfaces of the teeth by the production of insoluble dextran. This deposit makes the defusion of acid formed locally away from the surface of the tooth more difficult and simultaneously prevents the penetration of neutralizing saliva components.

Since saccharose is the only substance that can function as a substrate for the formation of plaque on the teeth a replacement of this sweetener in the food is one of the most important measures against caries. Comprehensive investigations have shown that even partial replacement of the saccharose has a positive effect.

Substituting maltose and/or glucose for saccharose thus constitutes an important step towards preventing caries. At least equally important in this context is the fact that maltose and maltotriose according to later investigations directly inhibit the formation of insoluble glucans (dextran) from saccharose with glucosyltransferase from Streptococcus mutans.

C) Quality-improving properties

The physical properties of different food-stuffs are to a large extent affected by the composition of the sweeteners which are present in the products. By a correct choice one may for example affect consistency, water evaporation, water absorption, colour, taste, storability and crystallisation.

Fructose is the most hygroscopic substance, then comes saccharose, glucose and lastly maltose. By choosing the correct mixture of glucose and maltose it is possible to control uptake as well as release of water to the desired level.

In order to increase the storability of the food-stuffs from a microbiological point of view a high osmotic pressure in the product is desirable. In this respect maltose is superior to saccharose, and a 40% maltose solution has equally great antibacterial effect as a 60% saccharose solution.

With the products prepared according to the present invention one has observed increased storability of bread and bakery products. The effect is obtained with a broad variation of the contents of maltose and glucose, in view of which the effect partly may be due to other components present, primarily proteins and higher oligosaccharides.

Detailed description of the invention

The process according to the present invention can be varied in view of the desired result according to the following:

I) The present invention suitably starts from flour of whole wheat including the fibre fraction if a final product having a high contents of fibre is desirable. The raw material may also be for example wheat flour having a degree of grinding of 70-80%.

Instead of wheat flour one may use for example barley flour from whole grains or flour sifted to a starch content of about 70%, but also flour from other cereals and starch products can be used, the fibre fraction being selected in dependence of the desired contents thereof in a final product.

The flour is admixed with ordinary water containing at least 50 ppm $Ca^{++}$. If the contents is lower a calcium salt is added so that the $Ca^{++}$-content becomes 40-150 ppm, preferably 70 ppm.

The quantity of water is adapted to the raw material, and the dry solids content (DS) is selected as high as possible by maintaining the desirable mobility of the suspension. A suitable DS is 30-50%, preferably 45%.

The pH suspension should lie between 5.0 and 7.0, prefeably 6.0. In most cases no adjustment of the pH of the prepared suspension will be required. Possible adjustment is made using hydrochloric acid and sodium hydroxide, respectively.

The enzymatic treatment is designed by considering the desired composition of the final product and the choice of starting raw material.

The initial enzymatic decomposition is provided by using alfa-amylase, for example some of the NOVO-enzymes BAN 120L, Fungamyl 800L or Termamyl 120L or a corresponding enzyme from another manufacturer. A suitable enzyme has been found to be Termamyl 120L.

The enzyme quantity used depends on the desirable period of incubation. 0.2-2.0 kg Termamyl 120L/ton starch is suitably added to the suspension, preferably 0.4-1.0 kg Termamyl/ton starch.

The enzyme is added to the suspension already at the temperature 20-40°C and then heating is performed with strong stirring to 80-95°C for a period of 1-3 hours. If a longer period of incubation is desired the quantity of enzyme used can be reduced to a corresponding extent.

The heating of the suspension may also take place in pressurized containers by direct injection of steam, the temperature being increased to 100-110°C, followed by a subsequent incubation in holding cells for 10-60 minutes.

The carbohydrate composition of the soluble fraction of the reaction mixture is continuously analyzed using HPLC as well as DS refractometrically. When the desired composition has been obtained the incubation is stopped.

pH is then lowered to 4.5 by adding hydrochloric acid under stirring, the temperature being maintained at 90-95°C for 5-30 minutes, preferably 10 minutes.

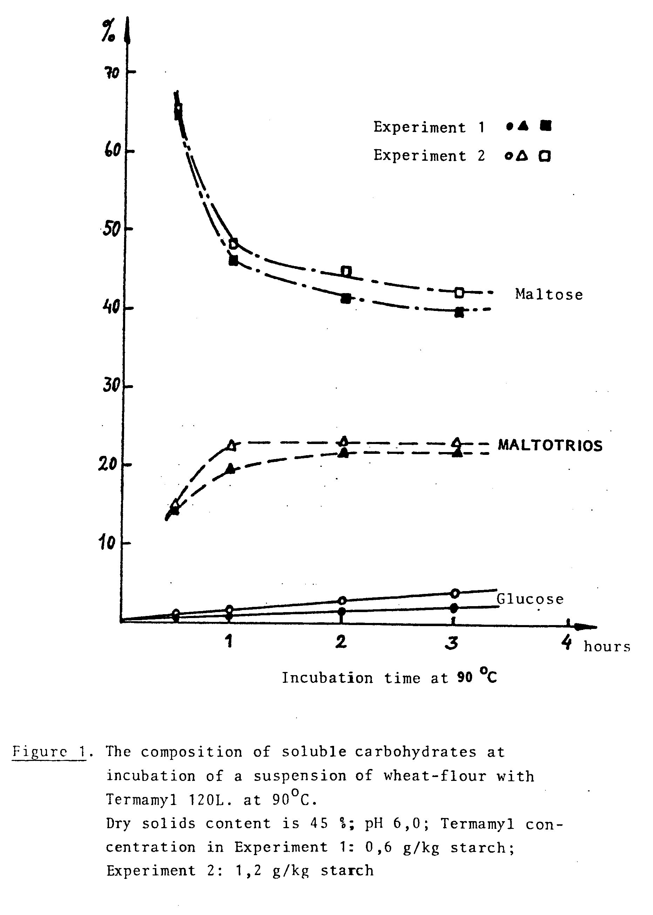

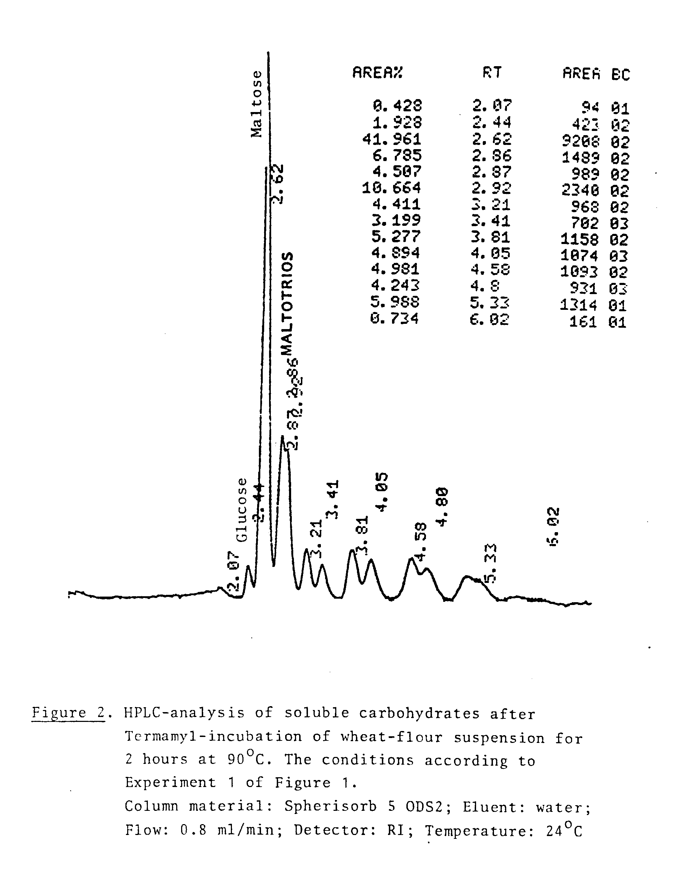

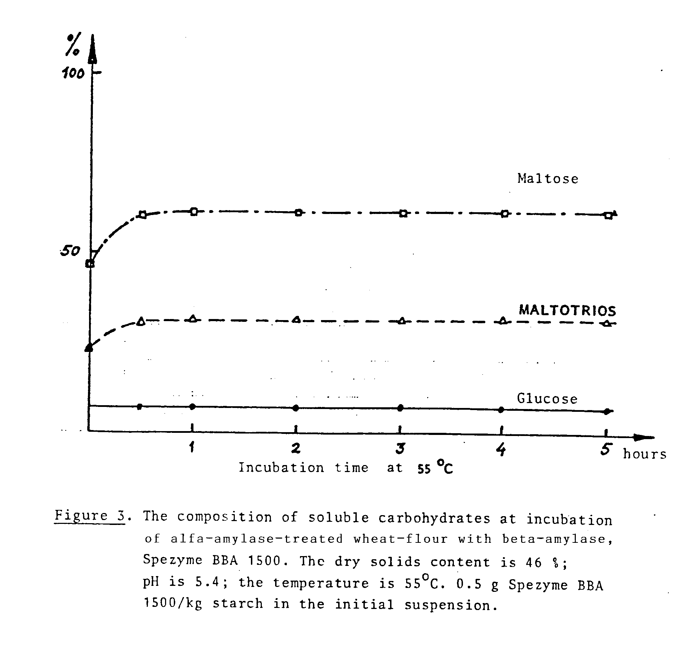

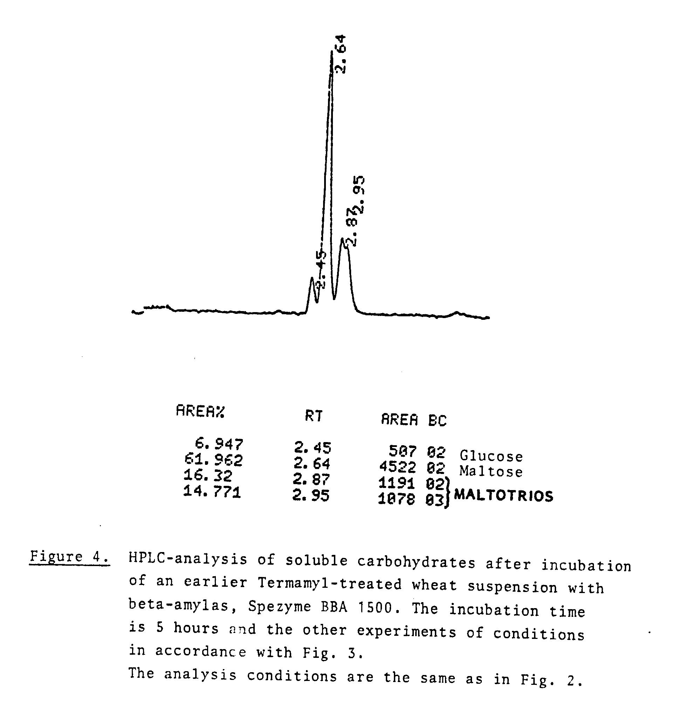

For wheat flour there will be obtained the results using different enzyme concentrations which are clear from Figs. 1 and 2. The suspension contains large quantities of maltose and maltotriose but also higher polymers and can be used directly since high concentrations of these components are desirable from the point of view of caries or for other reasons.

The suspension may also be used directly in baking etc., whereby also the contents of fibre and protein will be recovered in the final product. For certain areas of use it may be advantageous to separate the fibre-protein fraction from the soluble components. The insoluble components can be used in several food-stuff products, whereas the solution mainly containing the carbohydrates can be used i.a. in drinks where the contents of maltose, maltotriose and receptor active components are of value.

As indicated introductorily the suspension can be concentrated to a DS of 70-80% after removal of the fibre-protein fraction by centrifugation. The soluble fraction may also be dried to a pulverulant product using a spray-drier or other hot procedure.

Also the fibre-protein fraction can be dried if desirable using a conventional fibre drier.

However, it is advantageous according to the present invention to use the reaction product directly without time-consuming and costly drying or evaporation procedures. Certain valuable vitamines, trace elements etc. are, furthermore, often damaged in drying.

As explained in the following the product may also be given a higher stability by pasturization and effective packaging.

Preservation by the addition of allowed agents therefor may also be an alternative.

II) If a desired content of maltose and maltotriose in the final product is desirable the treatment described above using alfa-amylase may be supplemented with a subsequent treatment for beta-amylase alone or in combination with pullulanase. The latter enzyme cleaves 1,6-alfa-bonds and increases the yield of the di- and tri-saccharides.

A suitable beta-amylase is Finnsugar Groups Spezyme BBA 1500 and a suitable pullulanase is Novos Promozyme 200L.

Before the subsequent enzyme treatment pH of the suspension is adjusted with hydrochloric acid to 5.0-6.0, preferably 5.4. The enzyme treatment involved takes place before the pH is lowered to 4.5 according to I) in order to inactivate the enzymes.

To the suspension there will now be added 0.1-0-6 kg Spezyme BBA/ton starch in the suspension and as a supplement there may be added 0.4-1.2 kg Promozyme/kg starch. Preferably there is used 0.2 kg Spezyme BBA 1500 and 0.8 kg Promozyme 200L per ton starch of the suspension, respectively.

After the recommended enzyme addition incubation is carried out at 55-60°C for 1-5 hours. The composition of the soluble carbohydrates is analyzed using HPLC.

A typical experiment using wheat flour is presented in Figs. 3 and 4.

As earlier all enzymes are inactivated by adding hydrocloric acid to pH 4.5 and heating to 90-95°C.

As in I) the final product may be used directly or after concentration etc. as in I).

III) Using a barley-based flour as a raw material there will be obtained in the same manner as with certain other raw materials a highly viscous suspension. According to the present invention there is therefore recommended pre-treatment with beta-glucanase, for example Novos Finizyme 200L, before or simultaneously with the initial Termamyltreatment according to I).

In this case the suspension according to I) is supplied with 0.4-2.0 kg Finizyme/ton starch of the suspension, preferably 0.6 kg/ton. Then the mixture is incubated at 40-60°C for 30-60 minutes before increasing the temperature to 80-95°C for the final Termamyl-treatment.

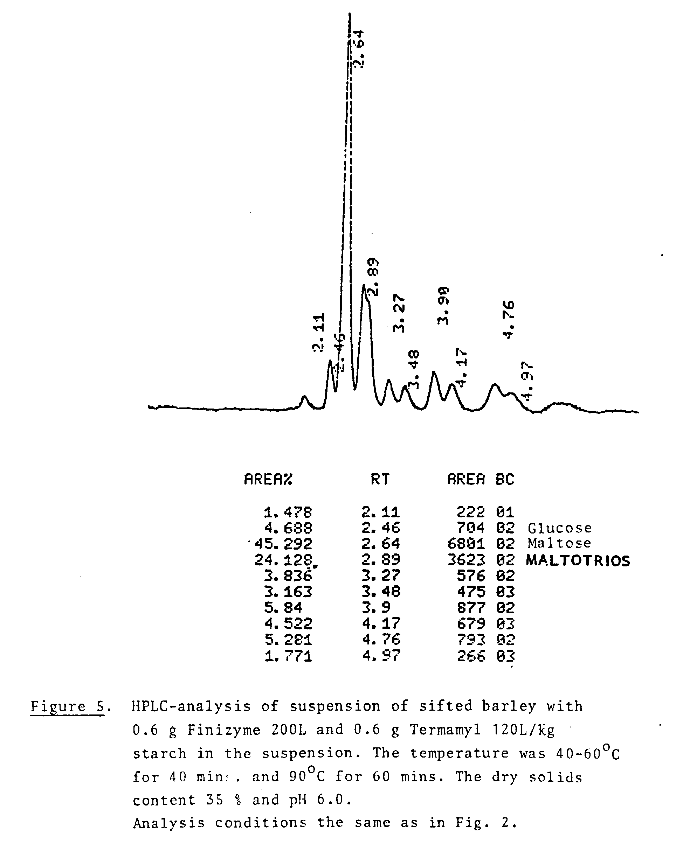

A typical experiment using both enzymes and barley flour is clear from Fig. 5.

IV) If a final product having a higher contents of glucos is desirable the enzyme-treatment according to I), II) or III) is followed by incubation with amyloglucosidase, for example Novos AMG 150L or Finnsugar Groups Spezyme GA 300.

The treatment with amyloglucosidase suitably takes place after the treatment with beta-amylase according to II) or simultaneously.

After the treatment with beta-amylase according to II) there is added to the suspension 0.1-2.5 kg AMG 200L/ton starch to the suspension, preferably 1.2 kg/ton. The mixture is incubated at unchanged pH and at 55-60°C for 1-8 hours.

The composition of the soluble carbohydrate fraction is analyzed continuously using HPLC, and the incubation is interrupted then the desired glucose content has been obtained. The enzymes are then rapidly inactivated by lowering pH to 4.5 using hydrochloric acid and heating to 90°C.

The treatment with beta-amylase and amyloglucosidase can also take place simultaneously. Then the enzyme quantities according to II) above are added together, whereafter the incubation takes place under the conditions given above as well as the subsequent inactivation of the enzymes.

As in III) pullulanase, for example Promozyme, can be added in order to increase the rate of conversion to glucose.

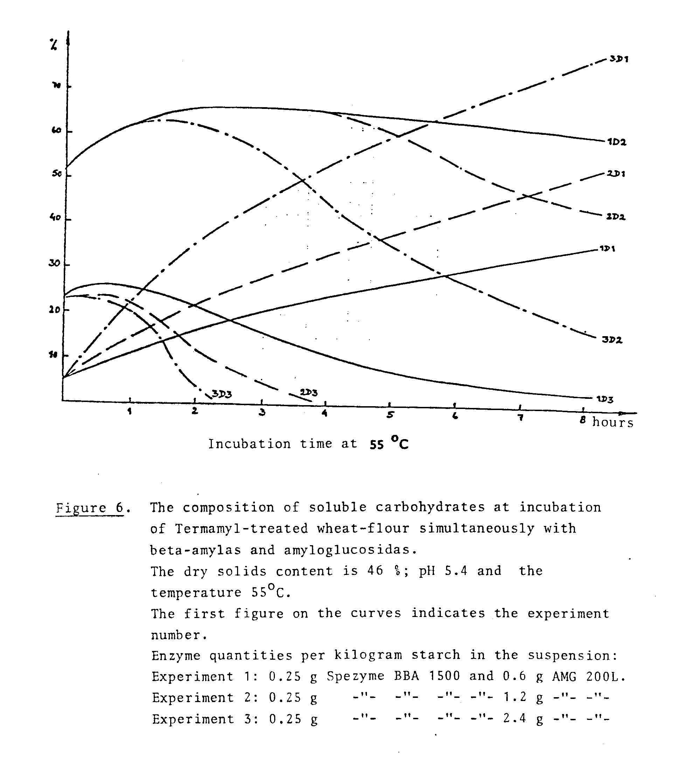

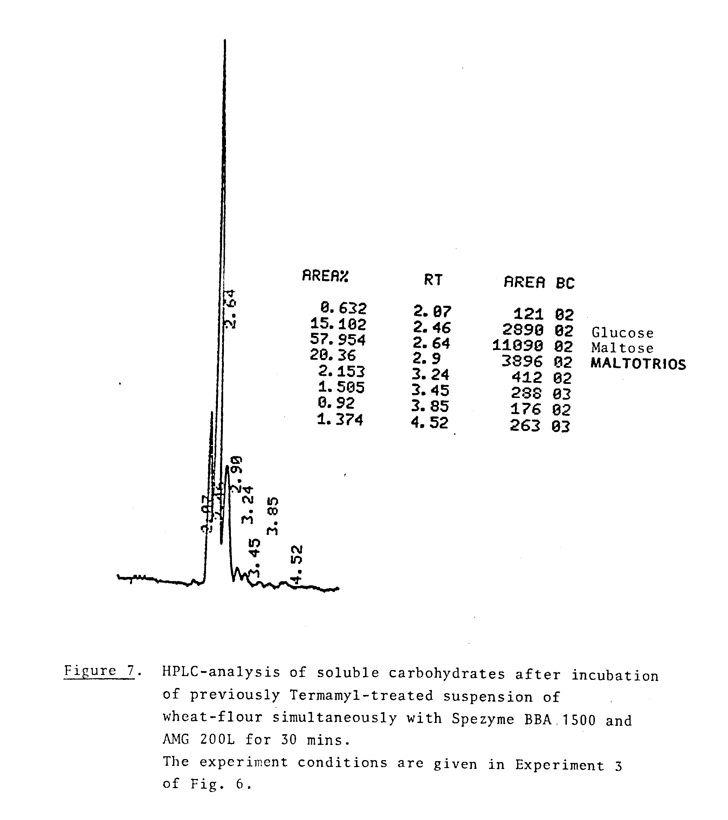

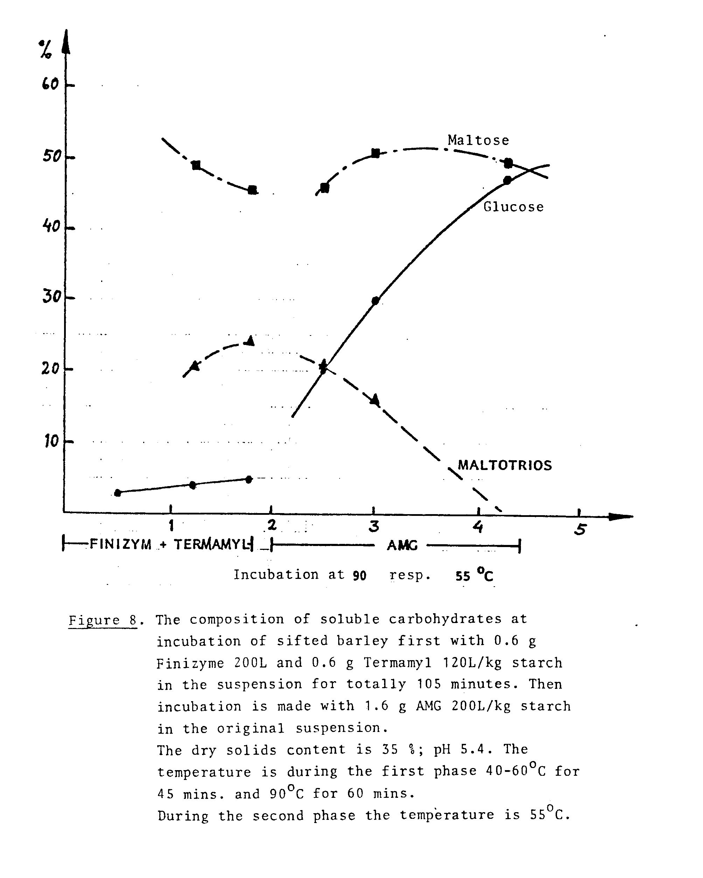

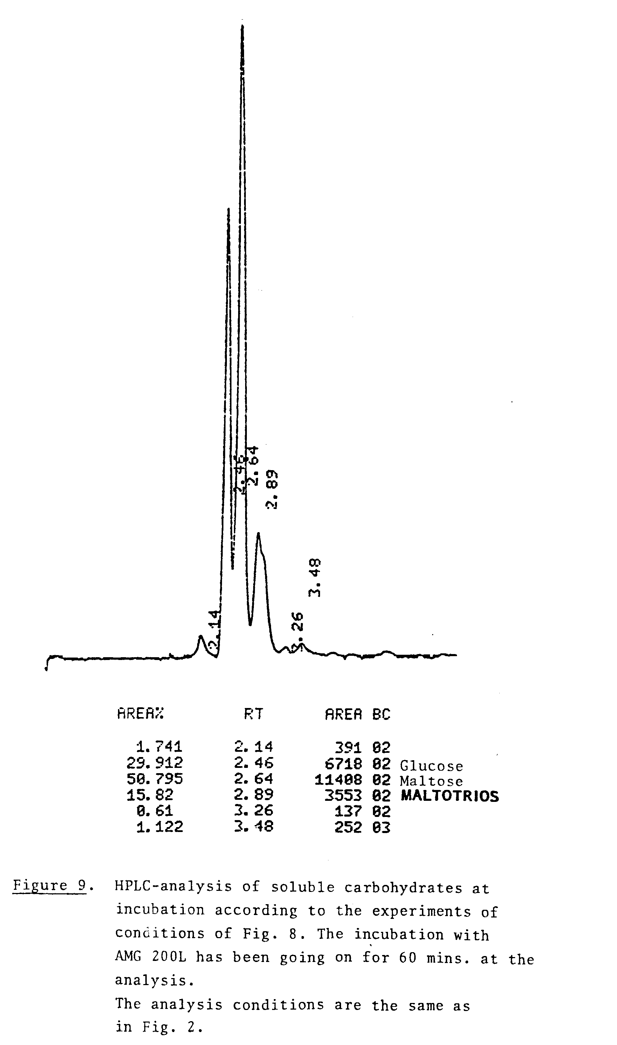

Representative experiments using wheat and barley flour is presented in Figs. 6-9.

V) The treatment with amyloglucosidase may also take place directly after the Termamyl treatment according to I), but then lower contents of all oligosaccharides are obtained.

VI) In order to avoid the difficulties of when operating on a large scale to quickly interrupt the enzyme treatment when the desired composition has been obtained the procedure may as an alternative be carried out so that two final products are obtained which then are admixed in suitable proportions to give a mixture of the desired composition.

A product A is prepared according to I), II) or III) containing a very little quantity of glucose and primarily maltose and maltotriose. The enzymatic conversion reaches certain maximum values and is then changed so slowly that the enzyme inactivation does not constitute any problem in relation to the composition.

A second product B of a high glucose content is then prepared according to IV). A glucose content of between 80 and 95% is desirable or in other words the glucose conversion must in this case proceed to a maximum value, whereafter the enzyme inactivation does not constitute any problem in this case.

By subsequent mixing A and B in suitable proportions a composition useful for any purpose will be obtained.

From a process point of view this process alternative involves clear advantages.

VII) The sweetness of the product can be increased by partial conversion of formed glucose to fructose by using glucoisomerase for example Novos Sweetzyme or Finnsugar Groups Spezyme IGI 450.

As a starting product there will suitably be used a suspension of high glucose content. Conventional procedures are used, and since these involve several ion-exchange chromatographic treatments only the water-soluble fraction of the suspension can be used.

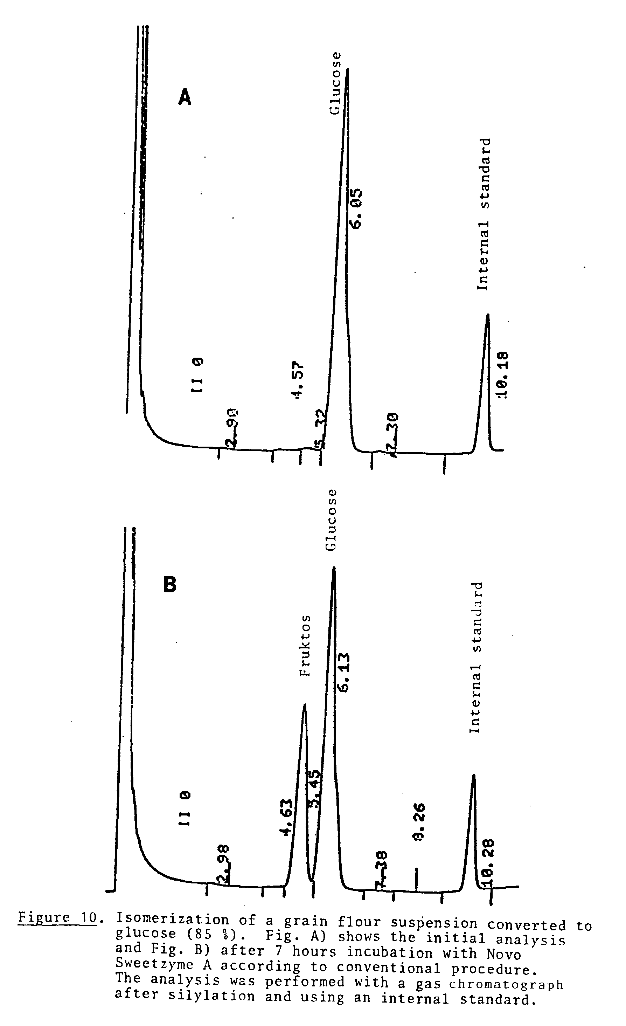

The isomerization does not involve any difficulties which is shown by Fig. 10 for a product based on barley flour. The process is used only exceptionally and as an alternative the sweetness can be increased by adding pure fructose, honey or even synthetic sweetener, for example Aspartame.

The areas of use and composition of the products

The products prepared according to the present invention have in view of their nutritionally physiological "plus-values" and functional properties many uses.

The products can be used not only as a replacement for saccharose, syrup etc. in view of their sweet taste or to add biologically active components in the form of specific receptors but also in order to impart to the food-stuff the desirable physical properties, such as

affecting consistency,

affecting the absorption or evaporation of water of the product,

increasing stability,

preventing crystallization of other components,

preventing or increasing taste, colour etc.

According to the present invention one can also prepare products which in regard to composition are taylor made for a certain purpose.

In this context one should not forget the positive properties that can be provided by the other components originating from the flour. It is primarily the proteins and the fibre components that can be considered to provide for clearly positive properties.

Some areas of use shall briefly be touched upon:

Baking

By adapting the raw material and the composition of the final product superior alternatives have been manufactured for saccharose as well as syrup.

For ordinary bread of the type syrup loaf there should be used a product based on wheat flour containing in the water-soluble fraction at most 25-30% maltose, whereas the contents of glucose can vary within wide limits. A higher glucose content results in higher sweetness and a more powerful formation of colour on the surface. This loaf contains in the normal recipe 300 g of household syrup/2 kg of flour and this can be replaced wholly by a product based on wheat flour containing for example 7% glucose and 22% maltose. At the same time the quantity of flour can be increased by 100 g resulting in unchanged volume of the final product.

Moreover, positive effects mainly due to present proteins in the form of better workability properties of the dough are observed. This effect is observed in all cases when products according to the invention are used even if smaller quantities are added.

When using the above type of bread the maltose content must not exceed the stated values if "tearing" or the formation of fissures shall be avoided.

For certain types of bread, for example French bread, fissures are desirable on the surface and this can be obtained by adding a product according to the above invention containing greater quantities of maltose, preferably 40-50%.

Full fermentation will be obtained in all baking uses independent of the variations in glucose and maltose contents.

In wheat bread often containing about 300 g sugar/2300 g flour, all of the sugar can advantageously be replaced with the corresponding quantity of the product according to the present invention containing for example 25% maltose and 70% glucose. The flour quantity can be reduced by about 5% and the final

7

product becomes equivalent with the corresponding product according to the original recipe. Also in this case better workability properties of the dough are observed.

From a nutritional-physiological point of view replacement of the saccharose in wheat bread with the above alternative constitutes important advantages. The somewhat lower sweetness of the wheat bread is wholly compensated by other ingredients in the final product.

Nutritional drinks

In the drink area there are many uses for products prepared according to the present invention.

The products directly prepared from different sorts of flour containing also insoluble fractions, fibres and proteins are mainly suited for powder drinks and such compositions where clear solutions are not compulsory.

Many new products can be developed based on the products according to the present invention and primarily nutritional drinks wherein the from nutritional-physiological point of view unsuited saccharose advantageously can be replaced.

By adapted enzymatic degradation the final product may, furthermore, provide for bacteriologically active receptors against oral as well as intestinal bacteria. The protein and fibre contents are also valuable in this connection.

Interesting combinations can be made for small children as well as adults. The products according to the present invention having a suitable carbohydrate composition, proteins and fibres supplemented with fruits and berries open new and valuable areas of use. The latter additions increase the vitamin as well as the fibre content and affect to a large extent the taste of the final product.

Aid foods are today based on cereal flour, leguminous plant flour and skim milk powder. The purpose of such food is mainly to supply proteins, energy, vitamins and minerals. There is possibility using the present invention not only to supply carbohydrates as energy donors but also as active components having the ability of affecting and normalizing the intestinal flora.

In this connection it is mainly products produced according to the above alternatives I)-III) which are of interest since they are rich in maltose, maltotriose and higher polymers of glucose, among which there are active receptors against bacteria.

For clear drinks there must be used products according to the present invention which have been freed from insoluble proteins and fibres by centrifugation. The contents of active receptor structures among the degradation products can still be used. The separated insoluble components can be used for supplementation both in food-stuffs and fodders.

Anticaries products

There is a need of producing products particularly directed towards caries prophylaxis, the presence of specific receptors against Streptococcus mutans and the positive effects shown with maltose and maltotriose being utilized.

In drinks as well as chewing gums these components may be added. The taste can be affected by adding flavouring substances from for example fruits and berries. If desired the sweetness can be increased with xylitol, aspartame or similar additives.

Other areas of use

The nutritional-physiological value of the products prepared according to the present invention motivates their use in many food-stuff products beyond those mentioned.

The advantage of replacing the saccharose is supplemented with other positive effects, not least the effect on the physical properties which can be obtained by playing with the specific properties of the main components maltose and glucose.

Interesting uses are found for ice-creams, jams, soft drinks, marmalades, confectionaries etc.

The invention will in the following be described by non-limiting examples.

EXAMPLE 1

Preparation of a mixture of maltose, maltotrios and higher glucose polymers using wheat flour as a raw material.

2000 g wheat flour (15% water and 12% protein) are mixed at 22°C with 2000 ml water containing 70 ppm $Ca^{++}$. To the suspension having a pH of 6.0 there is added 1.0 ml (1.2 g) Termamyl 120L.

The reaction mixture is maintained under stirring at 95°C on a water bath for 3 hours.

HPLC-analysis shows that the soluble carbohydrates consist i.a. of 2% glucose, 42% maltose and 22% maltotrios.

Refractometric drysubstance-determination (DS) shows 46.3%.

The mixture is used in Example 2 in view of which no deactivation of the enzyme is performed.

EXAMPLE 2

Preparation of a glucose-richer product using wheat flour as a raw material.

The reaction mixture from Example 1 is cooled to 55°C and the pH is then adjusted to 5.4 by the addition under stirring of dilute hydrochloric acid.

Then there is added 0.4 ml Spezyme BBA 1500 and 2.0 ml (2.4 g) AMG 200L. The reaction mixture is maintained at 55°C under stirring for 7 hours, dilute hydrochloric acid then being added for adjusting the pH to 4.5.

The mixture is heated in a microwave-oven to 95°C and is then allowed to cool at room temperature.

HPLC-analysis shows that the soluble carbohydrates now consist i.a. of 72% glucose and 22% maltose. DS is still 46%.

The mixture of the above composition has been prepared for use in the baking experiment described in Example 3.

EXAMPLE 3

Baking of a syrup loaf using the product from Example 2.

To evaluate the advantages of the product according to the present invention a so called syrup loaf is baked using the basic recipe and with the alternative wherein the household syrup has been replaced with the product from Example 2 containing 72% glucose and 22% maltose. A low maltose content was chosen to avoid "tearing".

|  | Base recipe | | Alternative recipe |
|---|---|---|---|
| Flour used | 2000 mg | | 1900 g |
| Water | 1000 ml | | 705 ml |
| Light household syrup | 300 g | Prod.ex. 2 | 550 ml |
| Yeast | 80 g | | 80 g |
| Salt | 30 g | | 30 g |
| Holdtime | 30 min. | | 30 min. |
| Volume of bread | 2400 ml | | 2400 ml |

The quantity of the reaction product according to Example 2 was chosen so that the quantity of glucose plus maltose corresponded to the quantity of mono- and di-saccharides in the syrup (DS 80%). The water quantity of the alternative recipe was also corrected while considering the water content of the reaction product from Example 2.

The two loafs of bread had equally large volume in spite of the fact that in the alternative recipe the quantity of flour had been reduced with 100 g.

The alternative product showed better dough workability properties in a farinograph as well as manually. The appearance of the loafs of bread was the same as well as other parameters of consideration disregarding a somewhat lower sweetness of the alternative product. Furthermore, the latter showed better durability in storage.

EXAMPLE 4

Preparation of glucose- and maltose-rich product using sifted barley as a raw material.

In the experiment sifted barley flour was used containing 70% starch, 8,5% protein and 11,5% water.

1500 g sifted barley was mixed at 22°C with 3000 ml water containing 70 ppm $Ca^{++}$. The suspension having a pH of 6.0 was supplied with 0.48 ml (0.63 g) Finizyme 200L and 0.53 ml (0.63 g) Termamyl 120L.

The mixture was placed on a water bath and with strong stirring the temperature is allowed to rise from 40-60°C for 45 minutes. Then, the temperature is rapidly increased to 90°C and incubation is continued for further 60 minutes.

The mixture is cooled to 55°C and the pH is adjusted to 5.4 by the addition under stirring of dilute hydrochloric acid. Then there are added 2.0 ml (2.4 g) AMG 200L and the whole is maintained under stirring on a water-bath at 55°C for 270 minutes.

Dilute hydrochloric acid for adjustment of the pH to 4.5 is added to the mixture under stirring, the mixture being then heated to 95°C in a microwave-oven and is then allowed to cool at room temperature.

HPLC-analysis shows that the soluble carbohydrates consist i.a. of 47% glucose and 50% maltose. The dry solid content is 35%.

EXAMPLE 5

Preparation of a product of higher contents of maltose and maltotriose using wheat flour as a raw material.

2000 g wheat flour (15% water and 12% protein) are mixed at 22°C with 2000 ml water containing 70 ppm $Ca^{++}$. The suspension having a pH of 6.0 is supplied with 1.0 ml (1.2 g) Termamyl 120L.

The reaction mixture is maintained under stirring at 95°C on a water-bath for 3 hours. Then the temperature is lowered to 55°C, whereafter the pH is lowered to 5.4 by the addition under stirring of dilute hydrochloric acid.

0.4 ml Spezyme BBA 1500 are then added. The reaction mixture is kept at 55°C with stirring for 3 hours, dilute hydrocloric acid for adjustment of the pH to 4.5 being then added under continued stirring.

The mixture is heated in a microwave-oven to 95°C and is then allowed to cool at room temperature.

HPLC-analysis shows that the soluble carbohydrates now consist of 4% glucose, 64% maltose, 30% maltotriose and 3% maltotetraose.

EXAMPLE 6

Preparation of a product more rich in fibre and having high contents of glucose and maltose using full grain flour of wheat as a raw material.

As a raw material there are used full grain flour of wheat ground from the whole wheat grain, i.e. grain, sprout and hull. The protein content is 11%, carbohydrate 72% of which food fibre is 8.5%.

2000 g full grain flour of wheat are mixed at 22°C with 4000 ml water containing 70 ppm $Ca^{++}$. To the suspension there is added under stirring a smaller quantity dilute hydrochloric acid for adjustment of the pH to 6.0.

Then there is added 1.0 ml (1.2 g) Termamyl 120L, whereafter the mixture is maintained under stirring at 95°C on a water-bath for 2 hours.

The mixture is cooled to 55°C, and under stirring there is added dilute hydrochloric acid for adjustment of the pH to 5.4, and then there are added 0.4 ml Spezyme BBA 1500 and 2.0 ml (2.4 g) AMG 200L.

The reaction mixture is maintained at 55°C under stirring for 4 hours, whereafter dilute hydrochloric acid is added for adjustment of the pH to 4.5.

The mixture is heated in a microwave-oven to 95°C and is then allowed to cool at room temperature.

The final product contains the high contents of the raw material of protein and fibre and according to HPLC-analysis a soluble carbohydrate fraction containing 45% glucose and 54% maltose.

EXAMPLE 7

Preparation of a product rich in fibre and with high contents of glucose and maltose using full grain flour of rye as a raw material.

As a raw material there is used full grain flour of rye containing 9% protein, 72% carbohydrates of which 13% are food fibres.

2000 g full grain flour of rye are mixed at 22°C with 4000 ml water containing 70 ppm $Ca^{++}$. To the suspension there is added under stirring a smaller quantity of dilute hydrochloric acid for adjustment of the pH to 6.0.

Then are added 1.0 ml(1.2 g) Termamyl 120L whereafter the mixture under stirring is maintained at 95°C on a water-bath for 2 hours.

The mixture is cooled to 55°C and under stirring there is added dilute hydrochloric acid for adjustment of pH to 5.4, and then there are added 0.5 ml Spezyme BBA 1500 and 1.2 ml (1.4 g) AMG 200L.

The reaction mixture is kept at 55°C with stirring for 15 hours, whereafter dilute hydrochloric acid is added for adjustment of the pH to 4.5.

The mixture is heated in a microwave-oven to 95°C and is then allowed to cool at room temperature.

The final product contains the proteins and fibres of the raw material and according to HPLC-analysis a soluble carbohydrate fraction containing 72% glucose and 24% maltose.

**Claims**

1.  Process for manufacturing food additives starting from whole flour of starch containing cereal grains, **characterized** in that the whole flour in an aqueous suspension having a dry solids contents of 30 to 50 % by weight, is, while maintaining a $Ca^{2+}$-ion level of 40 to 150 ppm in the suspension, subjected first to an enzymatic degradation using an alfa-amylase at 80 to 95°C and a pH of 5,0 to 7,0 while agitating the suspension for a period of 1 to 3 hrs, secondly that the suspension is subjected to a further enzymatic degradation using a beta-amylase at 55 to 60°C and a pH of 5.0 to 6.0, whereupon the pH of the suspension is adjusted to 4.5, the suspension is concentrated.

2.  Process according to claim 1, **characterized** in that the suspension is subjected to a further enzymatic degradation prior to the alfa-amylase treatment, using a beta-glucanase at 40 to 60°C for 30 to 60 min.

3.  Process according to claim 1, **characterized** in that the suspension is subjected to a further enzymatic degradation using an amyloglucosidase following or simultaneously with the beta-amylase treatment.

4.  Process according to claims 1-3, **characterized** in that the cereal flour is selected from the group of wheat, barley, rye, oats, rice, maize and sorghum flours.

**Patentansprüche**

1.  Verfahren zur Herstellung von Nahrungsmitteladditiven, ausgehend von Gesamtmehl stärkehaltiger Getreidekörner, **dadurch gekennzeichnet,** daß das Gesamtmehl in einer wäßrigen Suspension mit einem Trockenfeststoffgehalt von 30 bis 50 Gew.-%, während in der Suspension ein $Ca^{2+}$-Ionengehalt von 40 bis 150 ppm aufrechterhalten wird, erstens einem enzymatischen Abbau unter Verwendung einer alpha-Amylase bei 80 bis 95 °C und einem pH-Wert von 5,0 bis 7,0 unter Rühren der Suspension während einer Zeitdauer von 1 bis 3 h unterzogen wird und zweitens die Suspension einem weiteren enzymatischen Abbau unter Verwendung einer beta-Amylase bei 55 bis 60 °C und einem pH-Wert von 5,0 bis 6,0 unterzogen wird, worauf der PH-Wert der Suspension auf 4,5 eingestellt und die Suspension konzentriert wird.

2.  Verfahren nach Anspruch 1, **dadurch gekennzeichnet,** daß die Suspension einem weiteren enzymatischen Abbau vor der alpha-Amylasebehandlung unter Verwendung einer beta-Glucanase bei 40 bis 60 °C während 30 bis 60 min unterzogen wird.

3.  Verfahren nach Anspruch 1, **dadurch gekennzeichnet,** daß die Suspension einem weiteren enzymatischen Abbau unter Verwendung einer Amyloglucosidase nach oder gleichzeitig mit der beta-Amylasebehandlung unterzogen wird.

**4.** Verfahren nach den Ansprüchen 1 bis 3, **dadurch gekennzeichnet,** daß das Getreidemehl aus der Gruppe Weizen-, Gerste-, Roggen-, Hafer-, Reis-, Mais- und Sorghum-Mehl ausgewählt wird.

**Revendications**

**1.** Procédé de fabrication d'additifs alimentaires en partant de farine entière de grains de céréales contenant de l'amidon, caractérisé en ce que la farine entière dans une suspension aqueuse ayant une teneur en matières solides sèches de 30 à 50 % en poids est, tout en maintenant un niveau d'ions $Ca^{2+}$ de 40 à 150 ppm dans la suspension, soumise d'abord à une dégradation enzymatique en utilisant une alpha-amylase à une température de 80 à 95°C et à un pH de 5,0 à 7,0 tout en agitant la suspension pendant une période de 1 à 3 heures, qu'en second lieu la suspension est soumise à une nouvelle dégradation enzymatique en utilisant une bêta-amylase à une température de 55 à 60°C et à un pH de 5,0 à 6,0, après quoi le pH de la suspension est ajusté à 4,5, et la suspension est concentrée.

**2.** Procédé suivant la revendication 1, caractérisé en ce que la suspension est soumise à une nouvelle dégradation enzymatique avant le traitement par l'alpha-amylase, en utilisant une bêta-glucanase à une température de 40 à 60°C pendant 30 à 60 minutes.

**3.** Procédé suivant la revendication 1, caractérisé en ce que la suspension est soumise à une nouvelle dégradation enzymatique en utilisant une amyloglucosidase après ou en même temps que le traitement à la bêta-amylase.

**4.** Procédé suivant l'une quelconque des revendications 1 à 3, caractérisé en ce que la farine de céréale est choisie dans le groupe comprenant les farines de blé, d'orge, de seigle, d'avoine, de riz, de maïs et de sorgho.

Figure 1. The composition of soluble carbohydrates at
incubation of a suspension of wheat-flour with
Termamyl 120L. at 90°C.
Dry solids content is 45 %; pH 6,0; Termamyl con-
centration in Experiment 1: 0,6 g/kg starch;
Experiment 2: 1,2 g/kg starch

Figure 2. HPLC-analysis of soluble carbohydrates after Termamyl-incubation of wheat-flour suspension for 2 hours at 90°C. The conditions according to Experiment 1 of Figure 1.
Column material: Spherisorb 5 ODS2; Eluent: water; Flow: 0.8 ml/min; Detector: RI; Temperature: 24°C

| AREA% | RT | AREA | BC |
|---|---|---|---|
| 0.428 | 2.07 | 94 | 01 |
| 1.928 | 2.44 | 423 | 02 |
| 41.961 | 2.62 | 9208 | 02 |
| 6.785 | 2.86 | 1489 | 02 |
| 4.507 | 2.87 | 989 | 02 |
| 10.664 | 2.92 | 2340 | 02 |
| 4.411 | 3.21 | 968 | 02 |
| 3.199 | 3.41 | 702 | 03 |
| 5.277 | 3.81 | 1158 | 02 |
| 4.894 | 4.05 | 1074 | 03 |
| 4.981 | 4.58 | 1093 | 02 |
| 4.243 | 4.8 | 931 | 03 |
| 5.988 | 5.33 | 1314 | 01 |
| 0.734 | 6.02 | 161 | 01 |

Figure 3. The composition of soluble carbohydrates at incubation of alfa-amylase-treated wheat-flour with beta-amylase, Spezyme BBA 1500. The dry solids content is 46 %; pH is 5.4; the temperature is 55°C. 0.5 g Spezyme BBA 1500/kg starch in the initial suspension.

15

| AREA% | RT | AREA BC | |
|---|---|---|---|
| 6.947 | 2.45 | 587 02 | Glucose |
| 61.962 | 2.64 | 4522 02 | Maltose |
| 16.32 | 2.87 | 1191 02) | |
| 14.771 | 2.95 | 1078 03) | MALTOTRIOS |

Figure 4. HPLC-analysis of soluble carbohydrates after incubation of an earlier Termamyl-treated wheat suspension with beta-amylas, Spezyme BBA 1500. The incubation time is 5 hours and the other experiments of conditions in accordance with Fig. 3.
The analysis conditions are the same as in Fig. 2.

| AREA% | RT | AREA | BC | |
|---|---|---|---|---|
| 1.478 | 2.11 | 222 | 01 | |
| 4.688 | 2.46 | 704 | 02 | Glucose |
| 45.292 | 2.64 | 6801 | 02 | Maltose |
| 24.128 | 2.89 | 3623 | 02 | **MALTOTRIOS** |
| 3.836 | 3.27 | 576 | 02 | |
| 3.163 | 3.48 | 475 | 03 | |
| 5.84 | 3.9 | 877 | 02 | |
| 4.522 | 4.17 | 679 | 03 | |
| 5.281 | 4.76 | 793 | 02 | |
| 1.771 | 4.97 | 266 | 03 | |

Figure 5. HPLC-analysis of suspension of sifted barley with 0.6 g Finizyme 200L and 0.6 g Termamyl 120L/kg starch in the suspension. The temperature was 40-60$^{\mathrm{o}}$C for 40 mins. and 90$^{\mathrm{o}}$C for 60 mins. The dry solids content 35 % and pH 6.0.
Analysis conditions the same as in Fig. 2.

17

Incubation time at **55 °C**

Figure 6. The composition of soluble carbohydrates at incubation
of Termamyl-treated wheat-flour simultaneously with
beta-amylas and amyloglucosidas.
The dry solids content is 46 %; pH 5.4 and the
temperature 55°C.
The first figure on the curves indicates the experiment
number.
Enzyme quantities per kilogram starch in the suspension:
Experiment 1: 0.25 g Spezyme BBA 1500 and 0.6 g AMG 200L.
Experiment 2: 0.25 g    -"-   -"-   -"-  -"- 1.2 g -"- -"-
Experiment 3: 0.25 g    -"-   -"-   -"-  -"- 2.4 g -"- -"-

18

| AREA% | RT | AREA BC | |
|---|---|---|---|
| 0.632 | 2.07 | 121 02 | |
| 15.102 | 2.46 | 2890 02 | Glucose |
| 57.954 | 2.64 | 11090 02 | Maltose |
| 20.36 | 2.9 | 3896 02 | **MALTOTRIOS** |
| 2.153 | 3.24 | 412 02 | |
| 1.505 | 3.45 | 288 03 | |
| 0.92 | 3.85 | 176 02 | |
| 1.374 | 4.52 | 263 03 | |

Figure 7.  HPLC-analysis of soluble carbohydrates after incubation
of previously Termamyl-treated suspension of
wheat-flour simultaneously with Spezyme BBA 1500 and
AMG 200L for 30 mins.
The experiment conditions are given in Experiment 3
of Fig. 6.

Figure 8. The composition of soluble carbohydrates at
incubation of sifted barley first with 0.6 g
Finizyme 200L and 0.6 g Termamyl 120L/kg starch
in the suspension for totally 105 minutes. Then
incubation is made with 1.6 g AMG 200L/kg starch
in the original suspension.
The dry solids content is 35 %; pH 5.4. The
temperature is during the first phase 40-60°C for
45 mins. and 90°C for 60 mins.
During the second phase the temperature is 55°C.

| AREA% | RT | AREA BC | |
|---|---|---|---|
| 1.741 | 2.14 | 391 02 | |
| 29.912 | 2.46 | 6718 02 | Glucose |
| 50.795 | 2.64 | 11408 02 | Maltose |
| 15.82 | 2.89 | 3553 02 | **MALTOTRIOS** |
| 0.61 | 3.26 | 137 02 | |
| 1.122 | 3.48 | 252 03 | |

Figure 9. HPLC-analysis of soluble carbohydrates at incubation according to the experiments of conditions of Fig. 8. The incubation with AMG 200L has been going on for 60 mins. at the analysis.
The analysis conditions are the same as in Fig. 2.

21

EP 0 231 729 B1

Figure 10. Isomerization of a grain flour suspension converted to glucose (85 %). Fig. A) shows the initial analysis and Fig. B) after 7 hours incubation with Novo Sweetzyme A according to conventional procedure. The analysis was performed with a gas chromatograph after silylation and using an internal standard.